# EUROPEAN PATENT SPECIFICATION

(11) **EP 3 682 856 B1**
(45) Date of publication and mention of the grant of the patent: **25.10.2023**
(21) Application number: 19207893.9
(22) Date of filing: 08.11.2019
(51) Int. Cl.: A61F 9/00, A61M 5/32

(54) **DEVICE SUITABLE FOR INTRAOCULAR ADMINISTRATION OF A SUBSTANCE, FOR EXAMPLE A MEDICATION, INTO A HUMAN OR ANIMAL EYE**
VORRICHTUNG GEEIGNET ZUR INTRAOKULAREN VERABREICHUNG EINER SUBSTANZ, ZUM BEISPIEL EINES MEDIKAMENTS, IN EIN MENSCHLICHES ODER TIERISCHES AUGE
DISPOSITIF APPROPRIÉ POUR L'ADMINISTRATION INTRA-OCULAIRE D'UNE SUBSTANCE, PAR EXEMPLE UN MÉDICAMENT, DANS UN OEIL HUMAIN OU ANIMAL

(30) Priority: 17.01.2019 NL 2022410
(43) Date of publication of application: 22.07.2020
(73) Proprietor: Sharpsight B.V., 6049 MG Herten (NL)
(72) Inventor: GONÇALVES, Arnaldo, 6049 MG HERTEN (NL)
(74) Representative: Algemeen Octrooi- en Merkenbureau B.V.

(56) References cited:
- WO-A1-2016/083669
- US-A1- 2010 152 646

## Description

The invention relates to a device suitable for intraocular administration of a substance, for example a medication, into a human or animal eye by means of a hypodermic needle, comprising a support element to be placed on the eye as well as directing means for orienting the hypodermic needle relative to the eye, the support element comprises a support surface to be placed on the eye, wherein the support surface defines an elliptical configuration, as defined in the preamble of claim 1.

Such intraocular administering device according to the preamble of claim 1 is disclosed in International patent application mo. WO2016/083669A1.

Until recently, intravitreal or intraocular injections, by which a substance, and more in particular a medication, is injected into a human or animal eye by means of a hypodermic needle, were only used in exceptional cases. Especially in the treatment of endophtalmitis, an infrequently occurring intraocular inflammation of the eye, antibiotics are often administered intravitreally.

Lately, after the discovery of a new generation of medicines, it has become possible in ophthalmology to use intravitreal injections for treating certain eye disorders, which until now could hardly be therapeutically treated, or only to a limited extent. The eye disorders that can thus be treated include: macula degeneration, vena occlusions, diabetes retinopathy, all kinds of macula oedema, neovascular glaucoma, some forms of ischemic eye disorders, etc.

In the past the eye is held in place by means of tweezers, and the medication is injected into the eye. The position where the hypodermic needle is to be inserted into the eye is usually determined on the basis of the ophthalmologist's visual assessment and experience. An hypodermic needle that is inserted at an incorrect position or at an incorrect angle may cause complications, such as intraocular haemorrhage, or needle damage to the eye lens, which may in turn cause cataracts, retinal detachment and the like.

As a solution to the above illustrated drawbacks the International patent publication no. WO2008/097072 proposes a device as described in the preamble above. The device according to WO2008/097072 provides a tool which makes it possible, using one instrument, to keep the eye stationary and which at the same time makes it easier to give the injection, which procedure will be carried out under identical, reproducible circumstances at all times in that case.

A drawback of the known device is that due to its construction, it is not always easy to be placed correctly under the eyelids (specially by children, people who suffer from blepharospasmus, or with very small eyelid crevice). Furthermore, although the device according to WO2008/097072 allows a stationary placement on the eye, the device as a whole is in contact with the surface, which kan be difficult to be placed by eyelid squeezers due to fear experienced by the patient.

The object of the invention is to provide an improved tool, which not only allows a better visual overview of the treatment region / eye, but can also limit irritation to the eye. To this end, the device according to the invention is defined as outlined in the characterizing part of claim 1, that is the elliptical configuration of the support element exhibits an intermittent elliptical configuration being formed of at least two elliptical oriented support element parts, each elliptical oriented support element part having an elongated extending contact surface structured for placement on the eye and wherein at least one of the elliptical oriented support element parts is provided with at least one bore passing through the elliptical oriented support element part and exiting in the contact surface thereof, through which bore the hypodermic needle can be passed.

With this embodiment the support element is not in contact over the whole elliptical configuration of the support surface with the eye, which reduces tissue contact and irritation and makes it easier to be placed even in an eye with a very small eyelid crevice. Additionally the intermittent elliptical configuration being formed of the at least two elliptical oriented support element parts allows for a better visual overview of the treatment region as the support element no longer obscures the treatment region wholly.

The latter advantage is further improved by providing at least one of the elliptical oriented support element parts with at least one bore passing through the elliptical oriented support element part and exiting in the contact surface thereof, as this allows the hypothermic needle to be inserted into the eye from a side of the device, thus not further obscuring the treatment region.

In an example, the intermittent elliptical configuration is formed of at least three elliptical oriented support element parts, whereas in another example the intermittent elliptical configuration is formed of at least four elliptical oriented support element parts.

Preferably but not exclusively, the elliptical oriented support element parts are positioned at equidistant positions along said elliptical configuration.

In another example the elongated extending contact surface of at least one of the elliptical oriented support element parts has an arc length, which differs from the arc lengths of the elongated extending contact surfaces of the other elliptical oriented support element parts, whereas in yet another more simple example the elongated extending contact surfaces of the elliptical oriented support element parts have identical circular arc lengths.

In another embodiment the elongated extending contact surfaces of each of the elliptical oriented support element parts form a spherical segment part. Herewith a configuration is obtained wherein the elongated extending contact surface of each of the elliptical oriented support element parts accurately conforms and rests (or abuts) on the spherical surface of the eye. Herewith it is avoided that any pressure lines or pressure points are generated during placement of the device on the eye and subsequent during the performance of the intraocular administration of the substance, for example a medication, into the eye. This reduces unnecessary trauma to the eye.

Furthermore the at least one of the elliptical oriented support element parts is provided with friction-increasing means so as to realize a stable position on the eye. Said friction-increasing means consist of one or more barbs provided on the at least one elliptical oriented support element part. In either example, the device is prevented from slipping away on the eyeball during handling by a doctor or physician whilst intraocular administering a substance, for example a medication, into a human or animal eye.

In order to be able to insert the hypodermic needle into the eye in an identical, reproducible manner when carrying out repeated treatments, the directing means comprise at least one bore formed in the support element, through which the needle can be passed and wherein an upright edge is provided partly round the bore defining a space, which upright edge is open at a side facing away from the support element. In this way the successive treatments will be carried out under identical circumstances, thus preventing unnecessary trauma to the eye, such as complications resulting from incorrect insertion of the needle, for example intraocular haemorrhage, needle damage to the eye lens, retinal detachment, etc.

In particular, as the upright edge is open at a side facing away from the support element, this allows a simple access and positioning of the syringe with the hypodermic needle through the bore and against the upright edge and allows the device to be used with other injection/needle devices than a standard syringe.

In particular the angle between the bore and the eye surface ranges between 70° and 96°. This enables a reproducible placement and insertion of the hypodermic needle into the eye and prevents unnecessary trauma to the eye or to the patient. Additionally by locating the bore at the cornea-sciera transition zone of the eye when the support element is positioned on the eye.

Furthermore, in order to obtain an excellent reproducibility of the successive treatments, the support element is provided with at least one orientation projection to be oriented against the edge of the limbus of the eye.

Another embodiment of the device according to the invention is characterised in that it comprises means arranged for keeping the eye open. Thus, such eye treatments can be carried out in an identical, reproducible manner, in particular using one device, thereby preventing all kinds of trauma to the eye - resulting from the use of two different devices for keeping the eye open and inserting the hypodermic needle - and the associated inconvenience to the patient.

According to a specific example, which makes it possible to keep the eye open in a simple and patient-friendly manner, said means are disposed on either side of the support element. Thus, each eyelid can be kept open in an effective manner, so that the patient's involuntary blinking his or her eyes will not interfere with the insertion of the hypodermic needle. Said means may comprise at least one spreading element to be placed against an eyelid.

To spread the eyelids in a more effective manner, the spreading element extends in the plane of the support element, whilst the spreading element may optionally have a curved shape.

The invention will now be explained in more detail with reference to a drawing, in which:
Figure 1 a cross section of an eye of a human being;
Figure 2a-2e show different views of a first and second embodiment of a device according to the invention;
Figure 3a-3d show different views of a third embodiment of a device according to the invention;
Figure 4 shows a fourth embodiment of a device according to the invention;
Figure 5a-5b shows two views of an implementation of a device according of the invention whilst performing the intraocular administration of a substance in an eye of a human being.

For a better understanding of the invention, corresponding parts shown in the figures will be indicated by the same numerals in the description of the figures below.

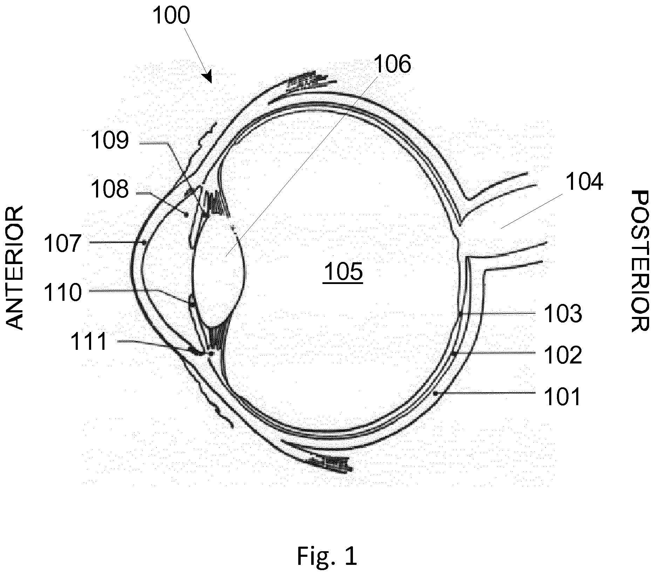

Figure 1 depicts a cross section of an eye, in particular a cross section of the eye 100 of a human being. The eye 100 is a sense organ capable of vision having an almost spherical configuration. Seen in the direction of light impinging on the eye, the anterior, frontal side of the eye 100 is formed by the cornea 107 having a stronger curvature comparted to the posterior side, being the sclera 101. The limbus connects the cornea 107 and the sclera 101. The cornea 107, the iris 110 and the lens capsule 106 form the anterior chamber 108 filled with aqueous humor liquid. Behind the lens 106 the vitreous body 105 is filled with vitreous gel.

The lens 106 is suspended in the eye by means of the ciliary body 111 composed of ciliary muscle and fibers. Reference numeral 109 denoted the posterior chamber present between the lens 106, the ciliary body 111 and the iris 110. The fundus or area opposite to the lens 106 at the posterior side of the eye 100 includes the macula 103, as well as the retina, being the innermost tissue layer containing the rod and cone cells. Also shown is the optic nerve 104 leaving the eye 100 at its posterior side towards the brains. Reference numeral 102 denotes the choroid.

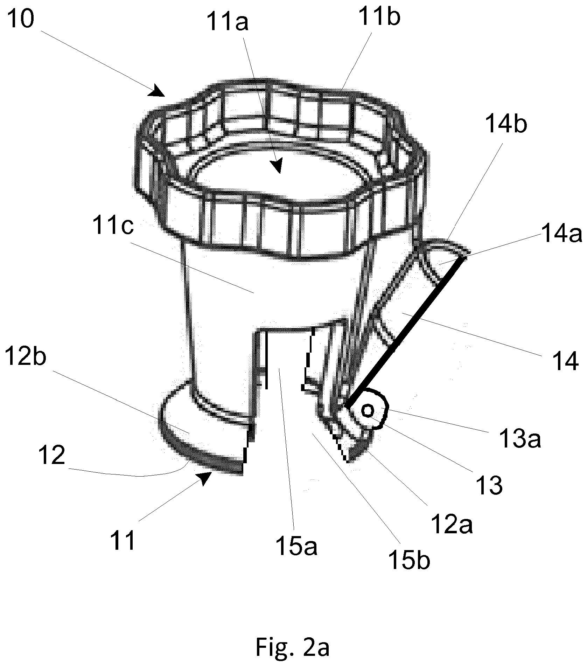

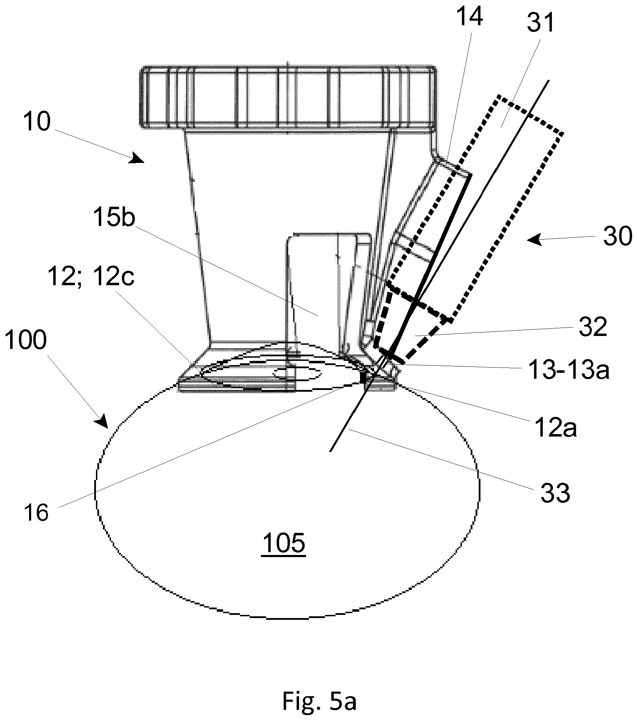

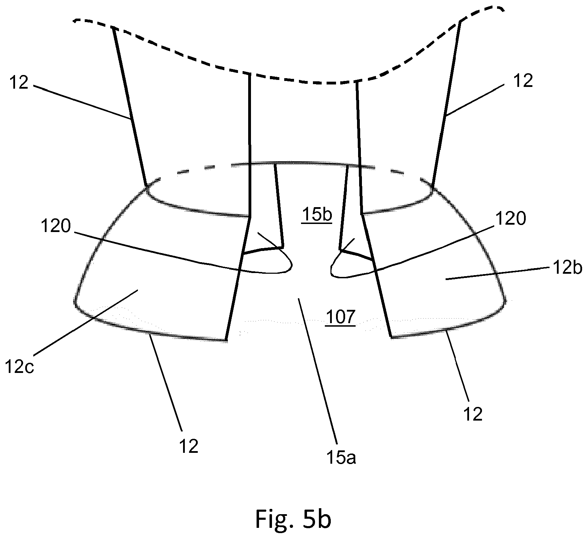

Figures 2a-2e show several views of a first embodiment a device according to the invention. The device 10 is made up of a support element 11, which is to be placed on the eye 100 (see figure 5). The support element 11 is provided with directing means 13-13a-14-14a-14b for orienting a dosing device 30 (again see Figure 5) relative to the eye 100.

The dosing device 30 can be any device capable of containing a substance, for example a medicine, and administering said substance in the eye 100. The dosing device can be a standard design syringe 31 provide with a hypothermic needle 33, which is provided with a Luer-Lok connector 32 for connection with the syringe 31.

However the device according to the invention suitable for intraocular administration of a substance, for example a medication, into the human or animal eye 100 can also be any other design of a container 31 for containing the substance provided with a needle 33 for insertion into the eye 100 for administering the substance.

In the figures 2a-2e the device 10 comprising a support element 11 to be placed on the eye as well as directing means 14-14a for orienting the dosing device 30 relative to the eye 100. The support element 11 comprises a support surface 12 to be placed on the eye 100. Preferably the support surface 12 defines an elliptical configuration, which means that the support surface 12 exhibits a circumference having the configuration of an ellipse, a circle, etc.

In the embodiments shown in the Figures the device 10 exhibits a support surface 12 having a circular circumference or a circular configuration. It should however be noted that the support surface 12 can be implemented in an annular or elliptical configuration.

As shown in Figures 2a-2e the elliptical (circular) configuration of the support element 12 exhibits an intermittent elliptical (circular) configuration being formed of at least two elliptical (circular) oriented support element parts 12a-12b. Because of the interrupted, intermittent elliptical (circular) configuration of the support surface being formed of two or more, here in the embodiments of Figures 2a-2e two, elliptical (circular) oriented support element parts 12a and 12b the contact area with the eye 100 is minimal, so that the patient is protected against trauma and/or irritation.

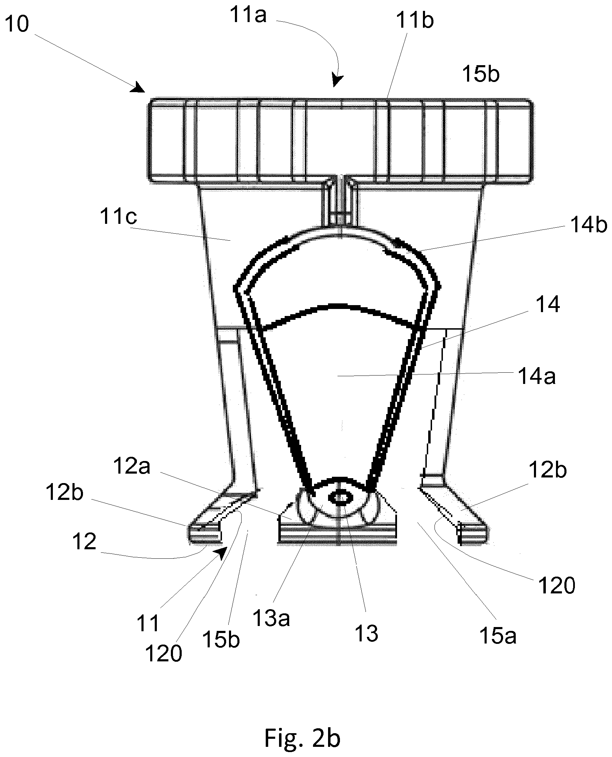

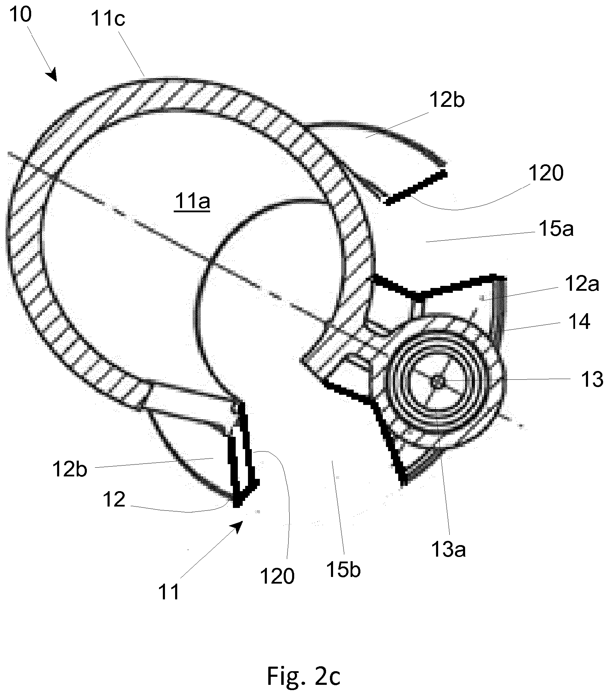

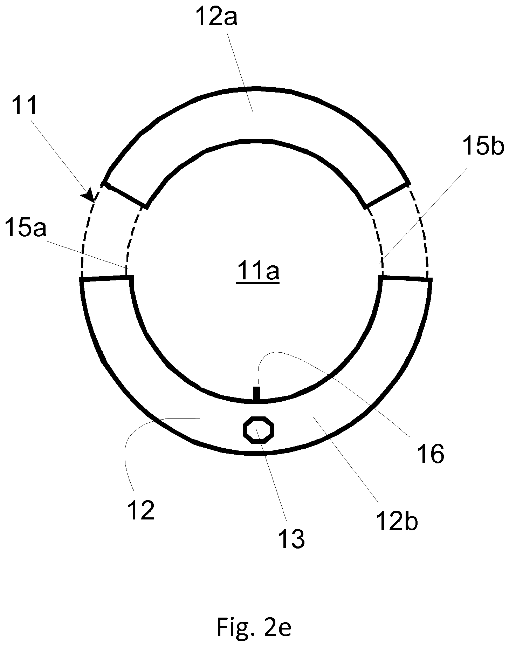

As depicted more clearly in Figure 2b and 2c, each elliptical oriented support element part 12a-12b has an elongated extending contact surface 120 structured for placement on the eye 105. Furthermore at least one of the elliptical oriented support element parts 12a-12b (in the example of Figure 2a and 2e the elliptical oriented support element part 12a) is provided with at least one bore 13, which bore 13 passes through the elliptical oriented support element part 12a and exits in the contact surface 120 thereof. Through the bore 13 a hypodermic needle can be passed.

Additionally, the creation of open access spaces 15a and 15b between the two elliptical (circular) oriented support element parts 12a and 12b allows for a better visual overview of the treatment region / eye 100 as the support element 11 no longer obscures the treatment region of the eye 100 wholly. With this embodiment the support element 11 is not in contact over the whole elliptical (circular) configuration of the support surface 12 with the eye 100, which reduces tissue contact and irritation.

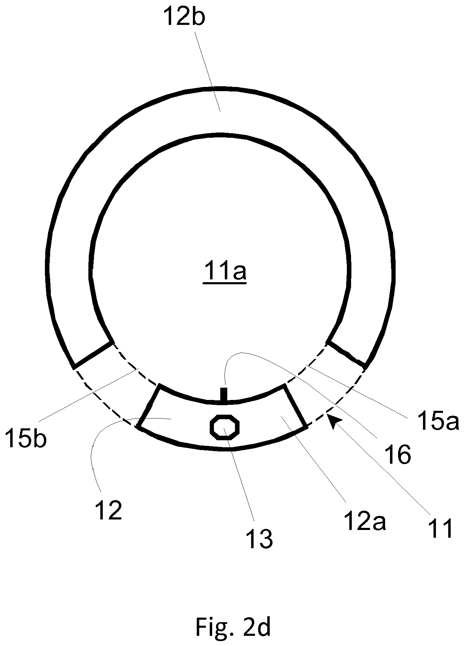

As shown in the views of Figures 2c and 2d the elongated extending contact surfaces 120 of the two elliptical oriented support element parts 12a and 12b of the support surface 12 have different (circular) arc lengths, the elongated extending contact surface 120 of the elliptical oriented support element part 12a having a significant shorter (circular) arc length than the (circular) arc length of the elongated extending contact surface 120 of the other elliptical oriented support element parts 12b.

Yet, in the embodiment shown in Figure 2e, the elongated extending contact surfaces 120 of the two elliptical oriented support element parts 12a and 12b still have different (circular) arc lengths, but the difference between them is significantly smaller than the embodiment of Figures 2c and 2d.

In another embodiment of the device 10 having a support surface 12 consisting of two elliptical oriented support element parts 12a and 12b, the (circular) arc lengths of the elongated extending contact surfaces 120 of both elliptical (circular) oriented support element parts 12a and 12b can be the same.

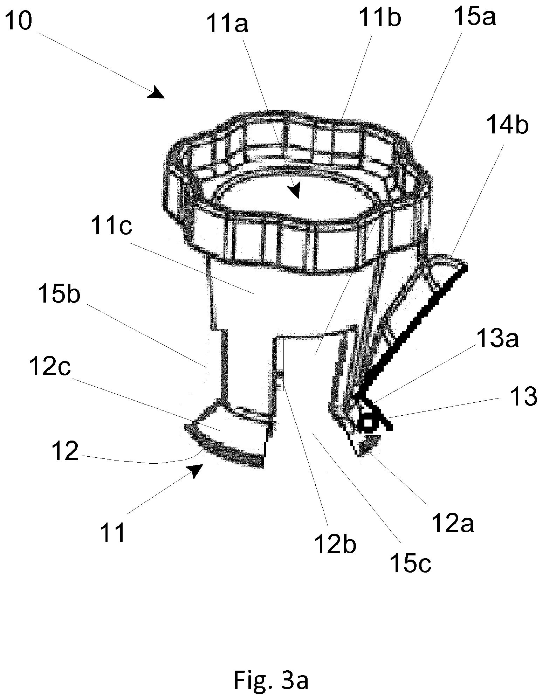

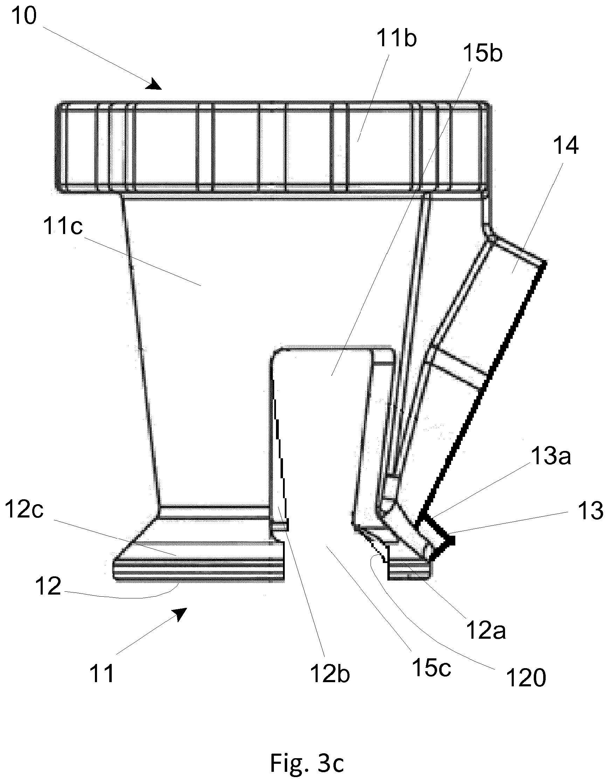

Figures 3a-3d show embodiments of a device 10 having a support surface 12 being composed of three elliptical (circular) oriented support element parts 12a-12b-12c.

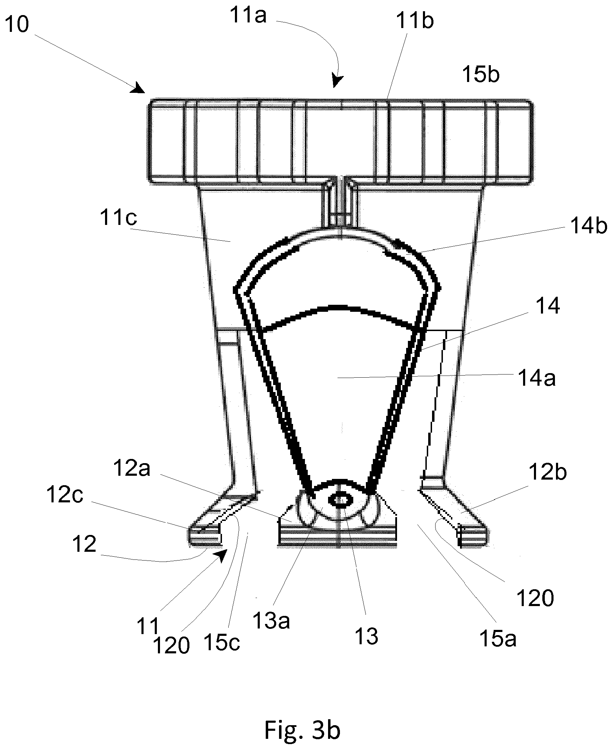

The embodiment of the device 10 as shown in Figures 3a-3c has a support surface 12 having a circular configuration and consisting of three elliptical oriented support element parts 12a-12b-12c and three open access spaces 15a-15b-15c. As shown in Figures 3a-3c, each elliptical oriented support element part 12a-12b-12c has an elongated extending contact surface 120 structured for placement on the eye 105. Furthermore at least one of the elliptical oriented support element parts 12a-12b-12c (in the example of Figure 3b and 3e the elliptical oriented support element part 12a) is provided with at least one bore 13, which bore 13 passes through the elliptical oriented support element part 12a and exits in the contact surface 120 thereof. Through the bore 13 a hypodermic needle can be passed.

In this embodiment the elliptical oriented support element part 12a exhibits an arc length being smaller than the arc lengths of the other two elliptical oriented support element parts 12b-12c, whose arc lengths can be (but not necessarily) identical in length.

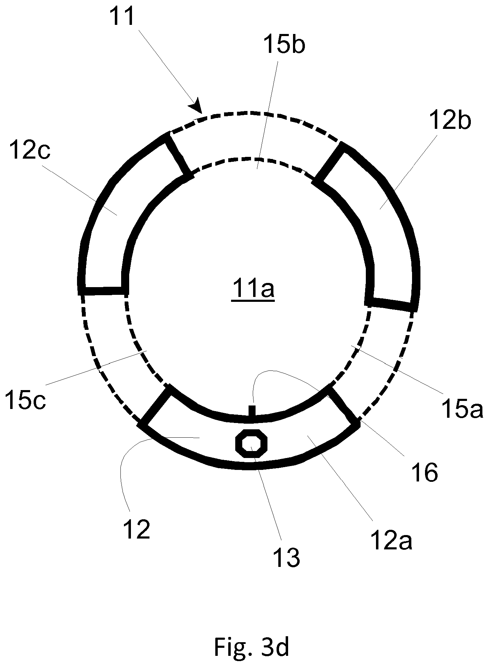

Also in the embodiment of Figure 3d the device 10 has a support surface 12 with a circular configuration and consisting three elliptical (circular) oriented support element parts 12a-12b-12c. In Figure 3d these three elliptical (circular) oriented support element parts 12a-12b-12c are positioned at equidistant positions along the elliptical (circular) configuration of the support surface 12. Also the arc lengths of the open access spaces 15a-15b-15c between the elliptical oriented support element parts 12a-12b-12c have, in this embodiment, identical (circular) arc lengths. Similarly the bore 13 passes through the elliptical oriented support element part 12a and exits in the contact surface 120 thereof. Through the bore 13 a hypodermic needle can be passed.

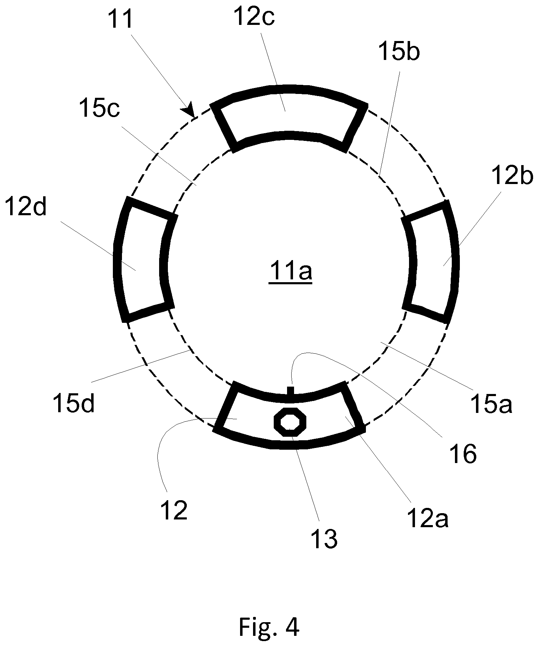

In Figure 4 a view of an embodiment of a device 10 is shown, with the support surface 12 having a circular orientation consisting of four elliptical (circular) oriented support element parts 12a-12b-12c-12d, which are positioned at equidistant positions along the elliptical (circular) configuration of the support surface 12. Also the arc lengths of the open access spaces 15a-15b-15c-15d between the elliptical oriented support element parts 12a-12b-12c-12d have, in this embodiment of Figure 4, identical (circular) arc lengths.

However it should be noted that in all embodiments shown in the several Figures the arc lengths of the several elliptical (circular) oriented support element parts as well as the open access spaces between these elliptical oriented support element parts can be arbitrary chosen, that is of the same or different arc lengths, being positioned equidistant or not along the elliptical (circular) configuration of the support surface 12.

All embodiments of the device 10 as shown and having different configurations of the elliptical support surface 12 consisting of two, three or four (or more) elliptical (circular) oriented support element parts having identical or different arc lengths also comprise directing means 14 for orientating the dosing device 30 and the hypodermic needle 33 relative to the eye 100.

The directing means 14 comprise at least one bore 13 formed in one of the elliptical (circular) oriented support element parts 12a of the support element 11. The bore 13 passes through the elliptical oriented support element part 12a and exits in the contact surface 120 thereof, and through which bore 13 a hypodermic needle 33 (of the dosing device 30 or syringe 31) can be passed. The bore 13 has been formed in the support element 11 in such a manner that the bore, in a first embodiment thereof, takes up a more or less perpendicular orientation relative to the eye surface and passes through the elliptical oriented support element part 12a and exits in the contact surface 120 thereof. In this way a reproducible placement of the device 10 on the eye 100, and thus an accurate insertion of the hypodermic needle 33 into the eye 100, is ensured at all times. This, too, prevents trauma to the eye or the patient, whilst furthermore preventing the risk of complications resulting from incorrect insertion or insertion at an incorrect angle of the hypodermic needle 33, such as intraocular haemorrhage, needle damage to the eye lens, retinal detachment, etc. The latter advantage is further improved by providing the bore 13 passing through the elliptical oriented support element part 12a and exiting in the contact surface 120 thereof, as this allows the hypothermic needle to be inserted into the eye 105 from a side of the device 10, thus not further obscuring the treatment region.

More in particular, the orientation of the bore 13 relative to the eye surface at all times will be identical at all times. The angle between the bore 13 and the eye surface is preferably more or less perpendicular and may optionally range between 70° and 95°.

Additionally the directing means 14 comprise an upright edge 14, which is bent or curved and provided partly round the bore 13. The bent, curved upright edge 14 defines a half-open space 14a, as the upright edge 14a is open at a side facing away from the support element 11 (device 10).

The half-open space 14a formed by the bent, curved upright edge 14 around the bore 30 allows the device 10 to be used with several types of dosing devices 30 having different designs and dimensions. The upright edge 14 together with the bore 13 makes it possible to easily and quickly position the dosing device 30 on the device 10 in repetitive, identical orientations relative to the eye 100 and to carry out repeated medical treatments, viz. inserting a substance in the eye 100.

In order to further improve the reproducibility of the medical procedure by means of the instrument 10 according to the invention, the device 10 is so arranged that when the support element 11 is positioned on the eye 100, the bore 13 will be spaced 3.5-4.5 mm from the cornea-sclera transition zone of the eye 100 at all times. This positioning is further supported and guaranteed in that the support element 11 is provided with at least one orientation projection 16 (Figures 2d-2e-3d-4-5), which extends on the inner side of the elliptical support surface 12 of the support element 11, and whose free end is to be placed or oriented against the edge of the limbus of the eye 100 in order to thus position the bore 13 at a distance of 3.5-4.5 mm from the cornea-sclera transition zone.

The diameter of the bore 13 is selected to accommodate 25G-30G hypodermic needles. This makes it possible to use the device 10 for various hypodermic needles 33 in dependence on the medical treatment to be carried out.

The reproducibility of the medical procedure, but above all the exclusion of unnecessary injury or trauma to the eye 100 can be realized in that an end edge 13a is provided around the bore 13, which end edge 13a functions as a stop for the hypodermic needle 33. This is clearly shown in Figure 5a, in which the hypodermic needle 33 is inserted into the eye 100 via the space 14a formed by the half-open bent, curved upright edge 14 and the bore 13, whilst the container/syringe 31 is positioned against the upright edge 14 and the Luer-Lok connector 32 is stopped by the end edge 13a.

The upright edge 14, which functions as a directing means for the dosing device / syringe 30-31 forms a half-open space 14a for allowing an easy accommodating of the dosing device / syringe 30-31 irrespective its outer dimensions, whilst numeral 14b indicates a top ridge of the half-open circular upright edge 14. The half-open space 14a merges into the bore 13, into which the hypodermic needle 33 can be inserted. The Luer-Lok connector 32 of the syringe 31 can be received in the space 14a and will be stopped by the end edge 13a.

In this way the hypodermic needle 33 can be inserted into the eye 100 to a reproducible, constant depth each time for administering a substance, such as a medication or medicine.

As shown in more detail in Figure 5b the elongated extending contact surfaces 120 of each of the elliptical oriented support element parts 12a-12b-12c form a spherical segment part. Herewith a configuration is obtained wherein the elongated extending contact surface 120 of each of the elliptical oriented support element parts 12a-12b-12c accurately conforms and rests (or abuts) on the spherical surface of the cornea 107 of the eye 105. Herewith it is avoided that any pressure lines or pressure points are generated during placement of the device on the eye 105-107 and subsequent during the performance of the intraocular administration of the substance, for example a medication, into the eye. Herewith it is prevented that unnecessary and undesired pressure is exerted in a concentrated manner in a small area on the eye surface (cornea) 107 and this reduces unnecessary trauma to the eye.

Additionally the device 10 can be provided with means (not shown) arranged for keeping the eye 100 open. More in particular, said means are provided on either side of the support element 11 and comprise a spreading element, which can be placed into abutment with each eyelid of the eye 100. The spreading element keeps the eye 100, more in particular both eyelids (not shown), of the eye 100 open. The spreading elements prevent the patient from blinking his or her eye involuntarily and thus disturb the treatment area for the ophthalmologist, resulting in incorrect insertion of the hypodermic needle 33 into the eye 100.

### LISTING OF REFERENCE NUMERALS

- 10: intraoccular device
- 11: support element
- 11a: inner space formed by support element
- 11b: top ridge of support element
- 11c: upright circular wall of support element
- 12: elliptical support surface configuration of the support element
- 12a-12b-12c: elliptical oriented support element parts
- 13: bore
- 13a: end edge around bore
- 14: directing means / upright edge
- 14a: inner space formed by upright edge of directing means
- 14b: top ridge of upright edge
- 15a-15b-15c: open access spaces between elliptical oriented support element parts
- 16: friction-increasing means or barbs
- 30: dosing device
- 31: container or syringe
- 32: Luer-Lok connector
- 33: hypodermic needle
- 100: eye
- 101: sclera
- 102: choroid
- 103: macula and retina
- 104: optic nerve
- 105: vitrous body
- 106: lens capsule (capsular bag)
- 107: cornea
- 108: anterior chamber
- 109: posterior chamber
- 110: iris
- 111: ciliary body
- 120: inner surface of spherical segment

## Claims

1. A device (10) suitable for intraocular administration of a substance, such as a medication, into a human or animal eye by means of a hypodermic needle (33), comprising a support element (11) to be placed on the eye as well as directing means (14) for orienting the hypodermic needle relative to the eye, the support element (11) comprises a support surface (12) to be placed on the eye, wherein the support surface defines an elliptical configuration, **characterized in that** said elliptical configuration of the support element (12) exhibits an intermittent elliptical configuration being formed of at least two elliptical oriented support element parts (12a-12b), each elliptical oriented support element part (12a-12b) having an elongated extending contact surface structured for placement on the eye and wherein at least one of the elliptical oriented support element parts (12a-12b) is provided with at least one bore (13) passing through the elliptical oriented support element part and exiting in the contact surface thereof, through which bore (13) the hypodermic needle can be passed.

2. A device according to claim 1, wherein the intermittent elliptical configuration is formed of at least three elliptical oriented support element parts (12a-12b-12c) or wherein the intermittent elliptical configuration is formed of at least four elliptical oriented support element parts (12a-12b-12c-12d).

3. A device according to claim 1 or 2, wherein the elliptical oriented support element parts are positioned at equidistant positions along said elliptical configuration.

4. A device according to any one or more of the claims 1-3, wherein the elongated extending contact surface of at least one of the elliptical oriented support element parts has a circular arc length, which differs from the circular arc lengths of the elongated extending contact surfaces of the other elliptical oriented support element parts or wherein the elongated extending contact surfaces of the elliptical oriented support element parts have identical circular arc lengths.

5. A device according to any one or more of the preceding claims, wherein the elongated extending contact surfaces of each of the elliptical oriented support element parts form a spherical segment part.

6. A device according to any one or more of the preceding claims, wherein at least one of the elliptical oriented support element parts is provided with friction-increasing means (16) so as to realize a stable position on the eye.

7. A device according to claim 6, wherein said friction-increasing means consist of one or more barbs (16) provided on the at least one elliptical oriented support element part.

8. A device according to any one or more of the preceding claims, wherein the directing means (14) comprise an upright edge provided partly round the bore (16) defining a space, which upright edge is open at a side facing away from the support element.

9. A device according to claim 8, wherein the angle between the bore and the eye surface ranges between 70° and 96°.

10. A device according to any one or more of the preceding claims, wherein the support element is provided with at least one orientation projection to be oriented against the edge of the limbus of the eye, such that the bore will be located at the cornea-sclera transition zone of the eye.

11. A device according to any one or more of the preceding claims, wherein said support element is provided with means arranged for keeping the eye open, which means are disposed on either side of the support element and comprise at least one spreading element to be placed against an eyelid extending in the plane formed by the support surface.

## Patentansprüche

1. Vorrichtung (10), die für die Intraokularverabreichung einer Substanz wie eines Medikaments in das Auge eines Menschen oder eines Tiers mittels einer Injektionsnadel (33) geeignet ist, umfassend sowohl ein Stützelement (11), das auf das Auge zu platzieren ist, als auch ein Ausrichtungsmittel (14) zum Ausrichten der Injektionsnadel bezüglich des Auges, wobei das Stützelement (11) eine Stützfläche (12) umfasst, die auf das Auge zu platzieren ist, wobei die Stützfläche eine elliptische Konfiguration definiert, **dadurch gekennzeichnet, dass** die elliptische Konfiguration des Stützelements (12) eine intermittierende elliptische Konfiguration aufweist, die aus mindestens zwei elliptisch ausgerichteten Stützelementteilen (12a - 12b) gebildet ist, wobei jeder elliptisch ausgerichtete Stützelementteil (12a - 12b) eine sich länglich erstreckende Kontaktfläche hat, die zur Platzierung auf dem Auge strukturiert ist, und wobei mindestens einer der elliptisch ausgerichteten Stützelementteile (12a - 12b) mit mindestens einer Bohrung (13) versehen ist, die durch den elliptisch ausgerichteten Stützelementteil geht und in der Kontaktfläche davon austritt, wobei die Injektionsnadel durch diese Bohrung (13) geführt werden kann.

2. Vorrichtung nach Anspruch 1, wobei die intermittierende elliptische Konfiguration aus mindestens drei elliptisch ausgerichteten Stützelementteilen (12a - 12b - 12c) gebildet ist oder wobei die intermittierende elliptische Konfiguration aus mindestens vier elliptisch ausgerichteten Stützelementteilen (12a - 12b - 12c - 12d) gebildet ist.

3. Vorrichtung nach Anspruch 1 oder 2, wobei die elliptisch ausgerichteten Stützelementteile an gleichbeabstandeten Positionen entlang der elliptischen Konfiguration positioniert sind.

4. Vorrichtung nach einem oder mehreren der Ansprüche 1 - 3, wobei die sich länglich erstreckende Kontaktfläche mindestens eines der elliptisch ausgerichteten Stützelementteile eine Kreisbogenlänge hat, die von den Kreisbogenlängen der sich länglich erstreckenden Kontaktflächen der anderen elliptisch ausgerichteten Stützelementteile verschieden ist, oder wobei die sich länglich erstreckenden Kontaktflächen der elliptisch ausgerichteten Stützelementteile identische Kreisbogenlängen haben.

5. Vorrichtung nach einem oder mehreren der vorhergehenden Ansprüche, wobei die sich länglich erstreckenden Kontaktflächen jedes der elliptisch ausgerichteten Stützelementteile einen Kugelsegmentteil bilden.

6. Vorrichtung nach einem oder mehreren der vorhergehenden Ansprüche, wobei mindestens einer der elliptisch ausgerichteten Stützelementteile mit Reibungserhöhungsmitteln (16) versehen ist, um eine stabile Position auf dem Auge zu realisieren.

7. Vorrichtung nach Anspruch 6, wobei die Reibungserhöhungsmittel aus einem oder mehreren Widerhaken (16) bestehen, die an dem mindestens einen elliptisch ausgerichteten Stützelementteil vorgesehen sind.

8. Vorrichtung nach einem oder mehreren der vorhergehenden Ansprüche, wobei die Ausrichtungsmittel (14) einen aufrechten Rand umfassen, der teilweise um die Bohrung (16) herum vorgesehen ist und einen Raum definiert, wobei der aufrechte Rand an einer von dem Stützelement weg weisenden Seite offen ist.

9. Vorrichtung nach Anspruch 8, wobei der Winkel zwischen der Bohrung und der Augenoberfläche zwischen 70° und 96° liegt.

10. Vorrichtung nach einem oder mehreren der vorhergehenden Ansprüche, wobei das Stützelement mit mindestens einem Ausrichtungsvorsprung versehen ist, der gegen den Rand des Limbus des Auges auszurichten ist, so dass die Bohrung an der Übergangszone zwischen Horn- und Lederhaut des Auges angeordnet ist.

11. Vorrichtung nach einem oder mehreren der vorhergehenden Ansprüche, wobei das Stützelement mit Mitteln versehen ist, die dazu angeordnet sind, das Auge offenzuhalten, wobei diese Mittel zu beiden Seiten des Stützelements angeordnet sind und mindestens ein Spreizelement umfassen, das gegen ein Augenlid zu platzieren ist und sich in der von der Stützfläche gebildeten Ebene erstreckt.

## Revendications

1. Dispositif (10) approprié pour une administration intraoculaire d'une substance, telle qu'un médicament, dans un oeil d'un être humain ou d'un animal au moyen d'une aiguille hypodermique (33), comprenant un élément de support (11) à placer sur l'œil ainsi que des moyens de direction (14) pour orienter l'aiguille hypodermique par rapport à l'œil, l'élément de support (11) comprend une surface de support (12) à placer sur l'œil, dans lequel la surface de support définit une configuration elliptique, **caractérisé en ce que** ladite configuration elliptique de l'élément de support (12) présente une configuration elliptique intermittente qui est formée d'au moins deux parties d'élément de support à orientation elliptique (12a-12b), chaque partie d'élément de support à orientation elliptique (12a-12b) ayant une surface de contact étendue allongée structurée pour être placée sur l'œil et dans lequel au moins l'une des parties d'élément de support à orientation elliptique (12a-12b) est pourvue d'au moins un alésage (13) traversant la partie d'élément de support à orientation elliptique et sortant dans sa surface de contact, à travers cet alésage (13) l'aiguille hypodermique peut être introduite.

2. Dispositif selon la revendication 1, dans lequel la configuration elliptique intermittente est formée d'au moins trois parties d'élément de support à orientation elliptique (12a-12b-12c) ou dans lequel la configuration elliptique intermittente est formée d'au moins quatre parties d'élément de support à orientation elliptique (12a-12b-12c-12d).

3. Dispositif selon la revendication 1 ou 2, dans lequel les parties d'élément de support à orientation elliptique sont positionnées à des positions équidistantes le long de ladite configuration elliptique.

4. Dispositif selon l'une quelconque ou plusieurs des revendications 1 à 3, dans lequel la surface de contact étendue allongée d'au moins l'une des parties d'élément de support à orientation elliptique a une longueur d'arc circulaire, qui diffère des longueurs d'arc circulaire des surfaces de contact étendues allongées des autres parties d'élément de support à orientation elliptique ou dans lequel les surfaces de contact étendues allongées des parties d'élément de support à orientation elliptique ont des longueurs d'arc circulaire identiques.

5. Dispositif selon l'une quelconque ou plusieurs des revendications précédentes, dans lequel les surfaces de contact étendues allongées de chacune des parties d'élément de support à orientation elliptique forment une partie de segment sphérique.

6. Dispositif selon l'une quelconque ou plusieurs des revendications précédentes, dans lequel au moins l'une des parties d'élément de support à orientation elliptique est pourvue de moyens augmentant la friction (16) de manière à réaliser une position stable sur l'œil.

7. Dispositif selon la revendication 6, dans lequel lesdits moyens augmentant la friction sont constitués d'une ou de plusieurs pointes (16) prévues sur l'au moins une partie d'élément de support à orientation elliptique.

8. Dispositif selon l'une quelconque ou plusieurs des revendications précédentes, dans lequel les moyens de direction (14) comprennent un bord vertical prévu en partie autour de l'alésage (16) définissant un espace, ce bord vertical est ouvert sur un côté opposé à l'élément de support.

9. Dispositif selon la revendication 8, dans lequel l'angle entre l'alésage et la surface de l'œil est compris entre 70° et 96°.

10. Dispositif selon l'une quelconque ou plusieurs des revendications précédentes, dans lequel l'élément de support est pourvu d'au moins une saillie d'orientation destinée à être orientée contre le bord du limbe de l'œil, de sorte que l'alésage soit situé au niveau de la zone de transition cornée-sclère de l'œil.

11. Dispositif selon l'une quelconque ou plusieurs des revendications précédentes, dans lequel ledit élément de support est pourvu de moyens agencés pour maintenir l'œil ouvert, ces moyens sont disposés de part et d'autre de l'élément de support et comprennent au moins un élément d'écartement à placer contre une paupière s'étendant dans le plan formé par la surface de support.
